**Europäisches Patentamt**

**European Patent Office**

**Office européen des brevets**

(19)

(11) Veröffentlichungsnummer: **0 192 181 B1**

(12) # EUROPÄISCHE PATENTSCHRIFT

(45) Veröffentlichungstag der Patentschrift: **02.05.91**

(51) Int. Cl.5: **A61F 5/04**

(21) Anmeldenummer: **86101779.6**

(22) Anmeldetag: **12.02.86**

(54) Abduktionsschiene zum Entlasten des Schultergelenkes.

(30) Priorität: **18.02.85 DE 3505528**

(43) Veröffentlichungstag der Anmeldung:
**27.08.86 Patentblatt 86/35**

(45) Bekanntmachung des Hinweises auf die
Patenterteilung:
**02.05.91 Patentblatt 91/18**

(84) Benannte Vertragsstaaten:
**AT BE CH DE FR GB IT LI NL SE**

(56) Entgegenhaltungen:
**DE-C- 582 933**
**US-A- 1 295 297**
**US-A- 3 952 733**
**US-A- 4 241 731**

(73) Patentinhaber: **Jacobsen, Uwe, Dr. med.**
**Albert-Mahlstedt-Strasse 10**
**W-2420 Eutin(DE)**

(72) Erfinder: **Jacobsen, Uwe, Dr. med.**
**Albert-Mahlstedt-Strasse 10**
**W-2420 Eutin(DE)**

(74) Vertreter: **Wilcken, Hugo, Dr. et al**
**Patentanwälte Dr. Hugo Wilcken Dipl.-Ing.**
**Thomas Wilcken Musterbahn 1**
**W-2400 Lübeck(DE)**

## Beschreibung

Die Erfindung betrifft eine Abduktionsschiene zum Entlasten des Schultergelenkes des betroffenen Armes eines Patienten nach dem Oberbegriff des Patentanspruchs 1, wie sie beispielsweise aus der US-A-4241731 bekannt ist.

Bei bekannten Abduktionsschienen dieser Art wird meist der überwiegende Teil der durch das Gewicht des Armes und der Abduktionsschiene bedingten Last im Bereich der Schulter vom Körper des Patienten aufgefangen, und zwar mittels eines um den Körper gelegten Gurtes, eines die Schulter umgreifenden Bügels und dergleichen. Der übrige Teil der Last wird mit gesonderten, im Bereich der Taille wirkenden Befestigungsmitteln auf den Körper des Patienten übertragen.

Zu diesem Zweck kann beispielsweise an der Stütze ein Schulterbügel angebracht sein (DE-U-79 15 687, US-A-4 241 731), der sich im Bereich der Schulter des Patienten abstützt und zusammen mit einem Hüftbügel mit Hilfe von Gürteleinrichtungen am Körper gehalten wird.

Eine andere Möglichkeit (DE-U-81 34 992, US-A-3 952 733) besteht darin, die Abduktionsschiene ohne Schulterbügel auszubilden und den größten Teil der Last über einen oder mehrere Gurte auf den Körper des Patienten einwirken zu lassen, während seitlich gerichtete Kippbewegungen der Schiene von Brust- und Schultergurten aufgefangen werden, wobei wenigstens der Schultergurt auch einen Teil des Gewichtes der Schiene und des Armes zu tragen hat. Wenn die Stütze von einer Hüftpelotte ausgehend eng am Körper nach oben verläuft, können die zum Körper gerichteten Gewichtskräfte auch mit einem Stützteil, z. B. mit einem Bügel oder einer Pelotte bzw. Platte, auf den Körper übertragen werden (DE-A-30 30 712), wobei der erwähnte Stützteil gegen eine Seite des Brustkorbes anliegt und diese für eine einwandfreie Funktion einer solchen Abduktionsschiene erforderliche Anlage normalerweise auch noch mit Brust- oder Schultergurten gesichert werden muß.

Abduktionsschienen der vorerwähnten Art haben den Nachteil, daß sie aufgrund der Anordnung der Befestigungsmittel im wesentlichen nur eine Ruhigstellung des betreffenden Armes zulassen, da eine Bewegung der Schiene relativ zum Patientenkörper nicht möglich oder weitestgehend eingeschränkt ist. Hieraus folgt, daß zwar eine an sich gewünschte Ruhigstellung des Armes mit Entlastung des Schultergelenkes gegeben ist, daß aber der Patient nicht in der Lage ist, mit angelegter Abduktionsschiene Bewegungen des Armes durch Schwenken, Anspreizen, Abspreizen und Anheben zu üben. Dies gilt vor allem dann, wenn die Bewegungsfreiheit des Armes und des Schultergelenkes durch Schulterbügel und Schultergurte eingeschränkt ist, wobei diese Befestigungsmittel außerdem noch besonders bei entwaigen Verletzungen des Schultergelenkes durch Druck Schmerzen verursachen.

Gewisse Probleme ergeben sich bei den bekannten Abduktionsschienen auch deshalb, weil die Patienten das Hantieren mit den Befestigungsmitteln bei An- und Ablegen der Schiene als mühsam empfinden oder gar nicht in der Lage sind, diese Tätigkeit ohne fremde Hilfe durchzuführen. Abgesehen hiervon machen die recht aufwendigen und häufig komplizierten Befestigungsmittel die Abduktionsschiene teuer.

Dementsprechend besteht die Aufgabe der Erfindung in der Schaffung einer einfach zu handhabenden und billig herstellbaren Abduktionsschiene, die einerseits eine optimale Ruhigstellung des Armes und des Schultergelenkes gewährleistet, andererseits aber dem Patienten auch die Möglichkeit gibt, weitgehend ungehindert und kontrolliert Armbewegungen zur Übung und zum Training durchführen zu können.

Die Lösung dieser Aufgabe ist in den Patentansprüchen und 2 angeführt.

Mit dieser Lösung sind die Freiheitsgrade für trainierende Armbewegungen erhöht, wenn zwischen den am Körper des Patienten befindlichen Befestigungsmitteln und der Armauflage zumindest ein Doppelscharniergelenk vorgesehen ist. Dabei kann der obere Gelenkkörper des Doppelscharniergelenkes unmittelbar an dem Unterarmteil der Armauflage angebracht sein. Die beiden parallelen Gelenkachsen am mittleren Gelenkkörper dieses Gelenkes sind exzentrisch zur Wirklinie der Stütze vorgesehen, und der untere Gelenkkörper ist mit der Stütze verbunden. Ein solches Scharniergelenk ermöglicht ein Verschwenken der Armauflage in zwei Richtungen. Das weitere, unten an der Schiene befindliche Gelenk erhöht weiter die Freiheitsgrade der Abduktionsschiene und damit die Übungsmöglichkeiten für den Patienten.

Wenn die Stütze als Stützstab mit beispielsweise teleskopisch ineinander eingreifenden Teilen ausgebildet ist, kann zwischen den beiden Stützstabteilen eine Feder angeordnet sein und so wirken, daß der obere, die Armauflage tragende Stützstabteil gegen und mit Wirkung der Feder axial und relativ zum unteren, hierbei feststehenden Stützstabteil bewegt werden kann. Dabei wird zweckmäßigerweise das eine Ende der Feder am beweglichen Stützstabteil und das andere Federende an einem Anschlag angreifen, der im feststehenden Stützstabteil axial verstellt und fixiert werden kann, um so die Länge der Stütze zwecks Anpassung der Abduktionsschiene an die Körpergröße des Patienten einstellen zu können. Hierdurch kann weiter ein Anheben der Armauflage relativ zur Stütze trainiert werden, wobei in diesem Fall der Arm mit Gurten an der Armauflage befestigtsein sollte.

Im übrigen wird die gesamte, von der Armauflage seitlich schräg nach unten zum Körper des Patienten gerichtete Last quasi nur an einem Punkt im Bereich der Taille auf den Körper des Patienten übertragen, wobei dieser Punkt im Prinzip als "Schwenklager" angesehen werden kann, daß bei willkürlichen Armbewegungen ein Verschwenken der Armauflagen nach vorn, hinten und zur Seite hin zuläßt, ohne daß hierbei irgendwelche, sonst bei den üblichen Abduktionsschienen im Bereich der Schulter und/oder des Brustkorbes vorgesehenen Befestigungsmittel stören bzw. solche Armbewegungen behindern. Des weiteren kann der Arm aber auch absolut ruhig gestellt bleiben, da er allein schon auf Grund der anatomischen Gegebenheiten in einer stabilen Lage von der Schiene sicher abgestützt wird, sofern der Patient nicht bewußt Armbewegungen üben will.

Die Erfindung ist nachstehend anhand mehrerer, in den anliegenden Zeichnungen dargestellter Ausführungsbeispiele näher erläutert. Es zeigen:

Figuren 1 bis 3
verschiedene Ansichten eines die vorgeschlagene Abduktionsschiene tragenden Patienten,
Figur 4
eine perspektivische Ansicht der erfindungsgemäßen Abduktionsschiene,
Figur 5 und 6
zwei Seitenansichten einer geteilten und in der Länge veränderbaren Stütze der Abduktionsschiene,
Figur 7
ein zwischen der Stütze und der Armauflage wirkendes Doppelscharniergelenk in perspektivischer Seitenansicht,
Figur 8
eine Seitenansicht eines Doppelscharniergelenkes mit relativ zueinander verstellbaren Gelenkachsen,
Figur 9
ein Doppelscharniergelenk mit durch Federn zusammengehaltenen Gelenkkörpern in Seitenansicht,
Figuren 10 und 11
eine weitere Ausführungsform für ein Gelenk der Abduktionsschiene.

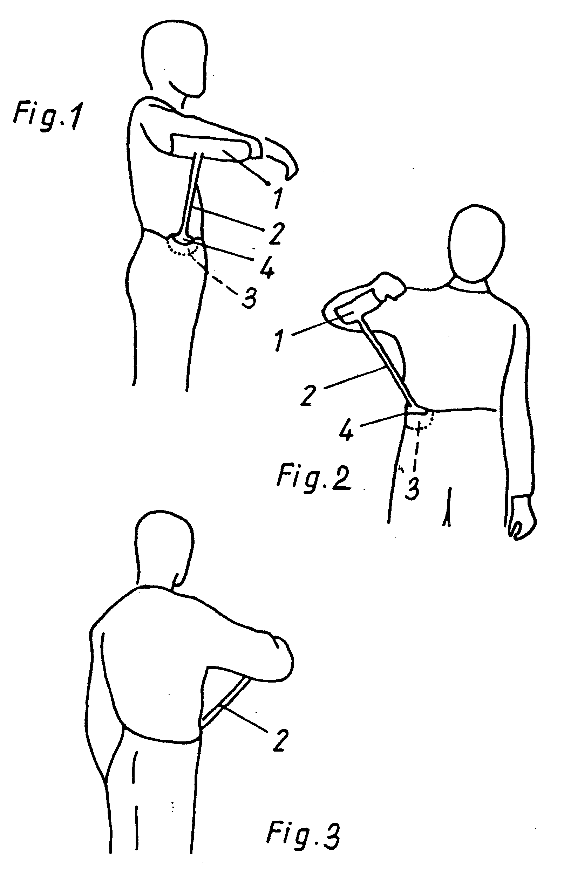

Die vereinfacht in den Figuren 1 bis 3 gezeigte Abduktionsschiene besteht hauptsächlich aus einer Armauflage 1 für den Unterarm, einer Stütze 2 in Form eines Stabes und aus am unteren Stützenende befindlichen Befestigungsmitteln, die bei diesem Beispiel aus einer Platte 3 mit einem Haken 4 gebildet sind. Die Platte 5 wird entsprechend den Figuren 1 bis 3 im Bereich der Taille des Patienten so von oben zwischen Hose und Hemd bzw. Unterbekleidung geschoben, daß der Haken 4 der Platte über den Gürtel oder den Hosenbund greift. Im übrigen kann die Platte auch in Anpassung an die Körperform des Patienten gewölbt sein, so daß sie beim Tragen nicht drückt und die einwirkende Stützkraft gleichmäßig auf den Anlagebericht am Körper verteilt.

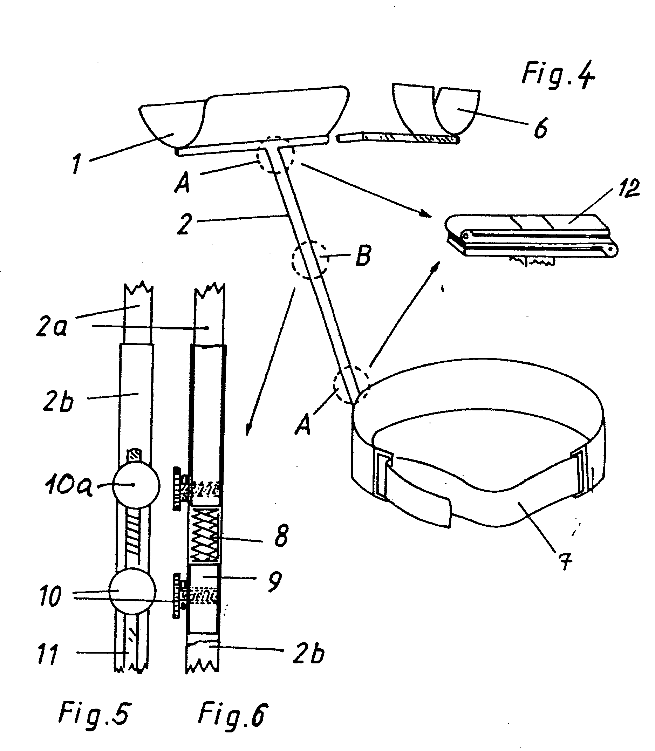

Bei der Abduktionsschiene nach Figur 4 besteht die Armauflage aus dem Unterarmteil 1 und einem Oberarmteil 6, während die unten an der Stütze 2 vorgesehenen Befestigungsmittel in an sich bekannter Weise aus einem verschließbaren Gurt 7 bestehen, der im Bereich der Taille um den Körper des Patienten gelegt wird. Auch in diesem Fall wird die durch den abgestützten Arm und die Abduktionsschiene bedingte Last wie beim vorher beschriebenen Beispiel im wesentlichen an einer Stelle seitlich auf den Körper übertragen.

An den mit den Kreisen A angedeuteten Stellen sind Scharniergelenke zwischen der Stütze 2 und der Armauflage 1 einerseits und dem Gurt 7 als Befestigungsmittel andererseits angeordnet, worauf weiter unten noch näher eingegangen ist.

Weiterhin kann die als Stützstab ausgebildete Stütze 2 an dem durch den Kreis B angegebenen Bereich unterteilt und wie in den Figuren 5 und 6 dargestellt ausgebildet sein, um eine Längenänderung der Stütze durchführen zu können. Die Bereiche A und B sind übrigens auch in dem Beispiel nach Fig. 1 finden, so daß auch das dort dargestellte Ausführungsbeispiel hinsichtlich der Gelenkausbildungen und einer Längenverstellung der Stütze in gleicher Weise ausgebildet sein kann, wie es nachfolgend im Zusammenhang mit den anderen Ausführungsformen beschrieben ist.

Eine Längenänderung der Stütze 2 kann so erreicht werden, daß den Figuren 5 und 6 entsprechend zwei Stützstabteile 2a, 2b beispielsweise teleskopisch ineinandergreifen und eine zwischen ihnen eingesetzte Druckfeder 8 so wirkt, daß der obere, die Armauflage 1, 6 tragende Stützstabteil 2a gegen und mit Wirkung dieser Feder axial und relativ zum unteren, hierbei feststehenden Stützstabteil 2b bewegt werden kann. Dabei greift das obere Ende der Feder 8 am unteren Ende des Stützstabteiles 2a und das untere Federende an einem Anschlag 9 an, der geführt im feststehenden Stützstabteil 2b verstellt und mit einer Schraube 10 am Stützstabteil 2b fixiert werden kann. Diese Schraube greift mit ihrem Gewindeschaft frei durch einen Längsschlitz 11 und ist in ein Gewindeloch des Anschlages 9 geschraubt, so daß beim Festdrehen der Schraube der Anschlag 9 mit der inneren Wandung des Stützstabteiles 2b verklemmt wird. Es ist offensichtlich, daß mit der jeweiligen axialen Position des Anschlages 9 die wirksame Länge der Stütze 2 eingestellt werden kann.

Mit einer Abduktionsschiene dieser Art kann der Patient das Anspreizen und Abspreizen des Armes üben, wobei die Feder 8 die Kraft bestimmt, die zum einen beim Anspreizen zu überwinden ist und zum anderen eine nachfolgende Abspreizbewegung unterstützen wird. Falls allerdings die beiden Stützstabteile 2a, 2b unter Ausschalten der Federwirkung starr verbunden werden sollen, wird eine weitere Schraube 10a angezogen, so daß der ebenfalls durch den Längsschlitz 11 verlaufende und im Stützstabteil 12a verschraubte Gewindeschaft diesen mit dem Stützstabteil 2b verklemmt.

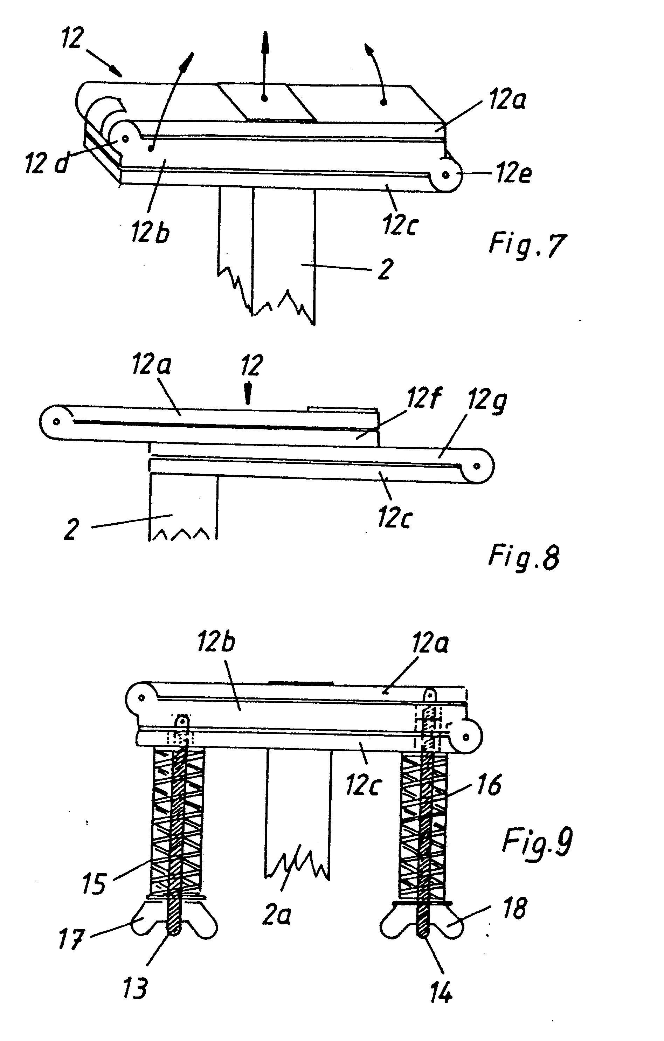

Wie bereits erwähnt ist, kann der Patient zur Durchführung von Bewegungsübungen den Arm ohne weiteres und ungehindert um ein gewisses Maß nach vorn und hinten bewegen, wobei die Armauflage 1 im wesentlichen auf einem Kreisbogen um das untere Ende der Stütze bzw. des Stützstabes 2 verschwenkt wird. Neben der weiteren und schon beschriebenen Möglichkeit des Anspreizens und Abspreizens des Armes ergeben sich weitere Freiheitsgrade für die Armbewegungen, dadurch, daß zwischen der Armauflage 1 und dem seitlich an der Taille befindlichen Befestigungspunkt zumindest ein Doppelscharniergelenk, vorgesehen ist, das eine Schwenkbewegung der Armauflage relativ zur Stütze 2 und/oder eine Schwenkbewegung der Stütze 2 relativ zu den Befestigungsmitteln 3, 7 zuläßt. Die Kreise A in Figur 7 deuten Stellen an, wo solche Scharniergelenke angeordnet sein können.

In Figur 7 ist ein Doppelscharniergelenk 12 gezeigt, daß am oberen Ende der Stütze 2 und vorzugsweise unmittelbar unter der nicht weiter dargestellten Armauflage 1 befestigt ist. Das Scharniergelenk besteht aus drei plattenförmigen Gelenkkörpern, und zwar aus dem oberen, starr an der Armauflage 1 befestigten Gelenkkörper 12a, aus dem mittleren Gelenkkörper 12b und dem unteren Gelenkkörper 12c, an dem unten die Stütze 2 starr befestigt ist. Im übrigen stehen diese Gelenkkörper über die beiden Achsen 12d und 12e so in Verbindung, daß die durch die Pfeile (Figur 7) angegebenen Bewegungen und Verstellmöglichkeiten gegeben sind. Der Gelenkkörper 12a kann also entgegengesetzt zum Uhrzeigersinn um die Achse 12d verschwenkt werden, während die Gelenkkörper 12a, 12b gemeinsam um die Achse 12e im Uhrzeigersinn verschwenkbar sind. Weiterhin kann der Gelenkkörper 12a in Parallellage zum feststehenden Gelenkkörper 12c angehoben werden. Schließlich können sich diese Verstellbewegungen je nach Richtung einer willkürlichen Armbewegung auch überlagern und in umgekehrter Richtung erfolgen, bis die Gelenkkörper wieder die dargestellte stabile Lage einnehmen. Diese Lage ist deshalb stabil, weil die beiden parallelen Gelenkachsen 12d, 12e exzentrisch zur Wirklinie der Stütze 2 liegen.

Die zum Aufklappen der Teile des Scharniergelenkes 12 erforderliche und von den Arm- sowie Schultermuskeln aufzubringende Kraft kann im übrigen auch in Abhängigkeit von der jeweils gewünschten Schwenkrichtung des Armes eingestellt werden, wenn die beiden Gelenkachsen relativ zueinander und/oder relativ zur Wirklinie bzw. gedachten Verlängerung der Stütze 2 verstellt werden. In diesem Fall kann der mittlere Gelenkkörper geteilt ausgebildet sein, so daß das Gelenk 12 gemäß Figur 11 aus zwei Einzelscharnieren mit je zwei Gelenkkörpern besteht, wobei die beiden mittleren, benachbarten Gelenkkörper 12f, 12g zwar miteinander verbunden sind, aber relativ zueinander verschoben werden können. Dies kann beispielsweise durch einen formschlüssigen Führungseingriff dieser beiden Gelenkkörper erreicht sein.

Die Kraft zum Aufklappen des Scharniergelenkes kann auch durch Federn beeinflußt und eingestellt werden. Hierzu zeigt die Figur 8 eine Lösung. Ein Gewindebolzen 13 ist durch ein Langloch des unteren Gelenkkörpers 12c geführt und am mittleren Gelenkkörper 12b angelenkt, während ein am oberen Gelenkkörper 12a angelenkter Gewindebolzen 14 durch Langlöcher der Gelenkkörper 12c, 12d verläuft. Auf diesen Gewindebolzen sitzen konzentrisch Druckfedern 15, 16, die sich nach der Darstellung mit ihren oberen Enden am Gelenkkörper 12c und mit ihren unteren Enden an Flügelmuttern 17, 18 abstützen (Fig. 9).

Mit den Flügelmuttern 17, 18 wird von Hand die Druckkraft eingestellt, mit der die Gelenkkörper zusammengehalten werden und die beim Üben von Armbewegungen von der Muskelkraft aufgebracht werden muß, um bei Schwenkbewegungen des Armes den Gelenkkörper 12a entgegengesetzt dem Uhrzeigersinn vom Gelenkkörper 12b oder diesen im Uhrzeigersinn vom Gelenkkörper 12c durch Verschwenken anheben zu können.

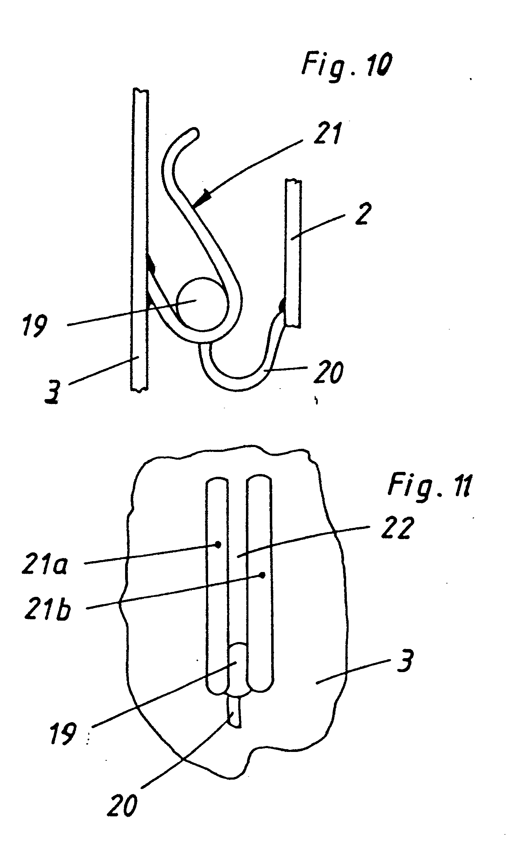

In den Figuren 10 und 11 ist eine Ausführungsform für ein Gelenk dargestellt, mit dem die Stütze 2 an ihrem unteren Ende mit der Platte 3 lösbar verbunden sein kann. Es handelt sich hierbei um ein Kugelgelenk, dessen Kugel 19 über einen Steg 20 mit der Stütze 2 verbunden ist. Die Kugel ruht derart in einer Mulde des an der Platte 3 befestigten Halters 21, daß die Stütze mit der Armauflage 1 relativ zur Platte 3 und somit zum Körper des Patienten verschwenkt werden kann. Der aus elastischem bzw. federndem Material bestehende Halter 21 hat zwei Arme 21a, 21b, zwischen denen ein oben offener Einführungsschlitz 22 ausgebildet ist, in den der Steg 20 paßt. Gemäß Figur 11 hat der Halter am oberen Ende einen Abstand zur Platte 3, der geringer als der Durchmesser der Gelenkkugel 19 ist.

Diese Gelenkverbindung wird so hergestellt, daß die Gelenkkugel 19 mit dem auf den Schlitz 22 ausgerichteten Steg 20 oben am Halter 21 angesetzt und nach unten gedrückt wird, so daß die Kugel unter Abspreizen des Halters von der Platte 3 in die erwähnte Mulde gelangt, wobei der Steg durch den Schlitz nach unten geführt wird.

Abschließend sei darauf hingewiesen, daß erforderlichenfalls auch der Oberarmteil 6 der Armauflage in seiner Position verstellt werden kann. Weiterhin besteht die Möglichkeit, an den Teilen 1 und 6 der Armauflage Gurte oder Bänder anzubringen, mit denen der Unter- und Oberarm auf den Armteilen 1 und 6 befestigt werden kann.

## Ansprüche

1. Abduktionsschiene zum Entlasten des Schultergelenkes durch Abstützen des betroffenen Armes in seitlich angehobener, vom Körper abgewinkelter Stellung mittels einer Armauflage (1), die gelenkig mit einer Stütze (2) verbunden ist und durch die Stütze getragen wird, deren Unterende verschwenkbar an einem am Körper des Patienten zu tragenden Abstützteil (3) befestigt ist, dadurch gekennzeichnet, daß die Stütze (2) an ihrem Oberende mit einem Doppelscharniergelenk (12) versehen ist, dessen oberer Gelenkkörper (12a) an der Armauflage (1) befestigt ist und dessen unterer Gelenkkörper (12c) mit der Stütze (2) verbunden ist, die an ihrem Unterende allseitig gelenkig mit dem am Körper des Patienten anlegbaren Abstützteil (3) verbunden ist.

2. Abduktionsschiene zum Entlasten des Schultergelenkes durch Abstützen des betroffenen Armes in seitlich angehobener, vom Körper abgewinkelter Stellung mittels einer Armauflage (1), die gelenkig mit einer Stütze (2) verbunden ist und durch die Stütze getragen wird, deren Unterende verschwenkbar an einem am Körper des Patieten zu tragenden Abstützteil (3) befestigt ist, dadurch gekennzeichnet, daß die Stütze (2) an ihrem Oberende mit einem Doppelscharniergelenk (12) versehen ist, dessen oberer Gelenkkörper (12a) an der Armauflage (1) befestigt ist und dessen unterer Gelenkkörper (12c) mit der Stütze (2) verbunden ist, und daß die Stütze (2) an ihrem Unterende mit einem weiteren Doppelscharniergelenk (12) versehen ist, das einerseits mit der Stütze (2) und andererseits mit dem am Körper des Patienten anlegbaren Abstützteil (3) verbunden ist.

3. Abduktionsschiene nach Anspruch 1 oder 2, dadurch gekennzeichnet, daß die beiden Gelenkachsen (12d, 12e) des Doppelscharniergelenkes (12) exzentrisch zur Wirklinie der Stütze (2) verlaufen.

4. Abduktionsschiene nach Anspruch 1 oder 2, dadurch gekennzeichnet, daß der mittlere Gelenkkörper (12b) jedes Doppelscharniergelenkes (12) aus zwei Gelenkkörpern (12f, 12g) zusammengesetzt ist, die relativ zueinander und/oder zur Wirklinie der Stütze (2) verstellbar sind.

5. Abduktionsschiene nach Anspruch 1 oder 2, dadurch gekennzeichnet, daß die Gelenkkörper (12a, 12b, 12c) durch Federkraft (15, 16) in zusammengeklappter Stellung gehalten werden.

6. Abduktionsschiene nach Anspruch 1, dadurch gekennzeichnet, daß die Stütze (2) an ihrem Unterende über ein Kugelgelenk (19, 20, 21) mit dem Abstützteil (3) verbunden ist.

7. Abduktionsschiene nach Anspruch 1 oder 2, dadurch gekennzeichnet, daß die Stütze (2) als Stützstab mit ineinander eingreifenden Teilen (2a, 2b) ausgebildet ist, daß zwischen den Stützstabteilen (2a, 2b) eine Feder (8) derart wirkt, daß der obere, die Armauflage (1) tragende Stützstabteil (2a) gegen die und mit der Wirkung der Feder axial und relativ zum unteren, hierbei feststehenden Stützstabteil (2b) bewegbar ist.

## Claims

1. An abduction splint for relieving the shoulder joint by supporting the arm in question in a laterally raised position angled away from the body by means of an arm rest (1) which is articulated to a support (2) and is carried by said support the lower end of which is pivotally secured on a carrying element (3) to be worn on the patient's body, characterized in that the support (2) is provided at its upper end with a double hinge joint (12) of which the upper joint member (12a) is secured to the arm rest (1) and of which the lower joint member (12c) is connected to the support (2) which at its lower end is connected by a universal joint to the carrying element (3) positionable against the body of the patient.

2. An abduction splint for relieving the shoulder joint by supporting the arm in question in a laterally raised position angled away from the body by means of an arm rest (1) which is articulated to a support (2) and is carried by said support the lower end of which is pivotally secured on a carrying element (3) to be worn on the patient's body, characterized in that the support (2) is provided at its upper end with a double hinge joint (12) of which the upper joint member (12a) is secured to the arm rest (1) and of which the lower joint member (12c) is connected to the support (2) and that the support (2) is provided at its lower end with another double hinge joint (12) which is connected on the one hand to the support (2) and on the other hand to the carrying element (3) positionable against the body of the patient.

3. An abduction splint according to Claim 1 or 2, characterized in that the two hinge axes (12d.12e) of the double hinge joint (12) extend eccentrically to the line of action of the support (2).

4. An abduction splint according to Claim 1 or 2, characterized in that the middle joint member (12b) of each double hinge joint (12) is a combination of two joint members (12f,12g) which are displaceable relative to each other and/or to the line of action of the support (2).

5. An abduction splint according to Claim 1 or 2, characterized in that the joint members (12a,12b,12c) are held in a closed position by means of spring force (15,16).

6. An abduction splint according to Claim 1, characterized in that the support (2) is connected to the carrying element (3) at its lower end via a ball-joint (19,20,21).

7. An abduction splint according to Claim 1 or 2, characterized in that the support (2) is constructed as a bearing rod comprising interengaged elements (2a, 2b), that a spring (8) acts between the bearing rod elements (2a,2b) in such a way that the upper bearing rod element (2a) carrying the arm rest (1) is movable against and with the action of the spring, axially and relative to the lower concomitantly stationary bearing rod element (2b).

**Revendications**

1. Attelle d'abduction servant à décharger l'articulation de l'épaule par soutien du bras concerné dans une position soulevée latéralement, obliquement par rapport au corps, à l'aide d'un appui-bras (1) qui est relié d'une manière articulée à un appui (2) et est porté par cet appui, et dont l'extrémité inférieure est fixée, de manière à pouvoir pivoter, à un élément de support (3) devant être porté sur le corps du patient, caractérisée en ce que l'appui (2) comporte, au niveau de son extrémité supérieure, une articulation à deux charnières (12), dont le corps articulé supérieur (12a) est fixé à l'appui-bras (1) et dont l'élément d'articulation inférieur (12c) est relié à l'appui (2), qui est raccordé d'une manière articulée de tous côtés, au niveau de son extrémité inférieure, à l'élément de support (3) qui peut être appliqué sur le corps du patient.

2. Attelle d'abduction servant à décharger l'articulation de l'épaule par soutien du bras concerné dans une position soulevée latéralement, obliquement par rapport au corps, à l'aide d'un appui-bras (1) qui est relié d'une manière articulée à un appui (2) et est porté par cet appui, et dont l'extrémité inférieure est fixée, de manière à pouvoir pivoter, à un élément de support (3) devant être porté sur le corps du patient, caractérisée en ce que l'appui (2) comporte, au niveau de son extrémité supérieure, une articulation à deux charnières (12), dont l'élément articulé supérieur (12a) est fixé à l'appui-bras (1) et dont l'élément d'articulation inférieur (12c) est relié à l'appui (2), et en ce que l'appui (2) comporte, au niveau de son extrémité inférieure, une autre articulation à deux charnières (12), qui est reliée d'une part à l'appui (2) et d'autre part à l'élément de support (3) qui peut être appliqué contre le corps du patient.

3. Attelle d'abduction selon la revendication 1 ou 2, caractérisée en ce que les deux axes d'articulation (12d,12e) de l'articulation à deux charnières (12) sont excentrés par rapport à la ligne d'action de l'appui (2).

4. Attelle d'abduction suivant la revendication 1 ou 2, caractérisée par le fait que le corps médian (12b) de chaque articulation à deux charnières (12) est formé par la réunion de deux corps d'articulation (12f,12g), qui peuvent être déplacés l'un par rapport à l'autre et/ou par rapport à la ligne d'action de l'appui (2).

5. Attelle d'abduction selon la revendication 1 ou 2, caractérisée en ce que les corps d'articulation (12a,12b,12c) sont maintenus dans la position repliée, par la force de ressorts (15,16).

6. Attelle d'abduction selon la revendication 1, caractérisée en ce que l'appui (2) est relié, au niveau de son extrémité inférieure, à l'élément de support (3) par l'intermédiaire d'une articulation sphérique (19,20,21).

7. Attelle d'abduction selon la revendication 1 ou 2, caractérisée en ce que l'appui (2) est réalisé sous la forme d'une barre d'appui comportant des éléments (2a,2b) venant en prise l'un dans l'autre, en ce qu'un ressort (8) agit entre les éléments de support (2a,2b) de telle sorte que l'élément de support supérieur (2a), qui porte l'appui-bras (1), peut être amené contre le ressort et être déplacé sous l'action de ce ressort axialement et par rapport à l'élément de support inférieur fixe (2b).

Fig.1

1
2
4
3

Fig.2

1
2
4
3

Fig.3

2

Fig.4

Fig.5    Fig.6

Fig.7

Fig.8

Fig.9

Fig. 10

21

2

19

3

20

Fig. 11

21a

22

21b

3

19

20